# EUROPÄISCHE PATENTSCHRIFT

(11) **EP 0 581 797 B1**
(45) Veröffentlichungstag und Bekanntmachung des Hinweises auf die Patenterteilung: **23.07.1997**
(21) Anmeldenummer: 92908327.7
(22) Anmeldetag: 01.04.1992
(51) Int. Cl.: C07D 409/12, A61K 31/41

(54) **NEUE THIOPHEN-2-CARBONSÄUREDERIVATE UND VERFAHREN ZU DEREN HERSTELLUNG**
NOVEL THIOPHENE-2-CARBOXYLIC ACID DERIVATIVES AND PROCESS FOR THEIR PRODUCTION
NOUVEAUX DERIVES D'ACIDE CARBOXYLIQUE DE THIOPHENE-2 ET LEUR PROCEDE DE PREPARATION

(30) Priorität: 04.04.1991 AT 716/91
(43) Veröffentlichungstag der Anmeldung: 09.02.1994
(73) Patentinhaber: Laevosan-Gesellschaft m.b.H., A-4020 Linz (AT)
(72) Erfinder: BINDER, Dieter, A-1190 Wien (AT); GREIER, Gerhard, A-4020 Linz (AT)
(74) Vertreter: Weinzinger, Arnulf, Dipl.-Ing.
(86) Internationale Anmeldenummer: AT9200044
(87) Internationale Veröffentlichungsnummer: WO9217472

(56) Entgegenhaltungen:
- EP-A- 0 109 381
- DE-A- 2 126 597
- GB-A- 2 065 121
- Grundlagen der Arzneimitteltherapie, W. DÖLL et al., B.I.-Wissenschaftsverlag, 1986, Seiten 81-83
- RÖMPP Chemie Lexikon, 9.Auflage, Bd.1, Georg-Thieme-Verlag 1989, Seiten 177 und 331

## Beschreibung

Die Erfindung betrifft neue therapeutisch wertvolle Thiophen-2-carbonsäurederivate und ein Verfahren zu deren Herstellung.

In der EP-A1-0 109 381 ist eine Verbindung der Formel (I') 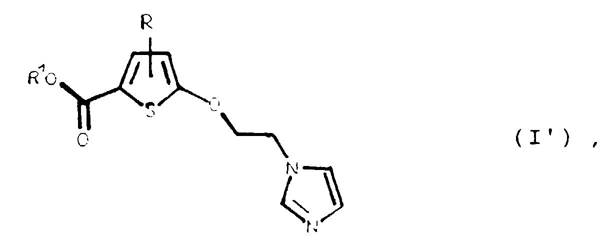 worin R in Stellung 3 oder 4 Wasserstoff, Methyl, Chlor oder Brom und R¹ Wasserstoff oder C₁-C₄-Alkyl bedeuten, mit starker Hemmwirkung auf die Thromboxansynthetase ohne signifikante Hemmung der Wirkung der Prostacyclinsynthetase- oder Cyclooxygenaseenzyme aus Thrombozytenmikrosomen geoffenbart.

Die Verbindung der Formel (I'), worin R¹ H bedeutet, weist bei oraler Verabreichung jedoch nur geringe Resorption auf.

Es wurde nun gefunden, daß die 1-Alkoxycarbonyloxyethylester der Verbindung der Formel (I') eine verbesserte Resorption bei oraler Verabreichung aufweisen, da sie nach Passage durch den Darm im Blut als freie Carbonsäure vorliegen und daher geeignete Arzneimittelvorläufer ("Prodrugs") der Verbindung der Formel (I') darstellen.

Die erfindungsgemäßen Verbindungen der im nachstehenden angegebenen Formel (I) wirken dadurch bei oraler Verabreichung auf die Thromboxansynthetase stark hemmend ohne signifikante Hemmung der Wirkung der Prostacyclinsynthetase- oder Cyclooxygenaseenzyme aus Thrombozytenmikrosomen, d.h. diese Verbindungen hemmen die Umwandlung von Prostaglandin-H₂ zu Thromboxan-B₂ über Thromboxan A₂, das ein instabiles Zwischenprodukt ist und das bekanntlich die irreversible Plättchenaggregation induziert und glatte Muskeln, insbesondere die der Blutgefäße, kontrahiert. Diese Tatsache zeigt, daß die Verbindungen der Formel (I) die Biosynthese von Thromboxan A₂ hemmen und demgemäß zur Behandlung von Krankheiten geeignet sind, die durch Thromboxan A₂ verursacht werden, wie z.B. Entzündung, Hypertonie, Thrombus, Gehirnschlag, Asthma, Angina pectoris, ischämische Herzleiden, ischämische Anfälle, Migräne und vaskuläre Komplikationen von Diabetes.

Gegenstand der vorliegenden Erfindung sind somit neue Verbindungen der allgemeinen Formel (I) 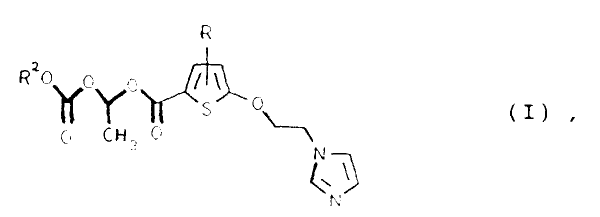 worin R in Stellung 3 oder 4 Wasserstoff, Methyl, Chlor oder Brom darstellt und R² C₁-C₁₀-Alkyl, C₃-C₇-Cycloalkyl oder Benzyl ist, und ihre pharmazeutisch verträglichen Additionssalze mit schwachen organischen Säuren.

Die Resorption der erfindungsgemäßen Verbindungen bei oraler Verabreichung beträgt mindestens das dreifache der Resorption bei oraler Verabreichung der Verbindungen gemäß EP-A1-0 109 381.

Ein weiterer Gegenstand der vorliegenden Erfindung ist ein Verfahren zur Herstellung der neuen Verbindungen der Formel (I), worin R und R² die oben angegebenen Bedeutungen haben, das darin besteht, daß ein Salz einer Verbindung der Formel (I") 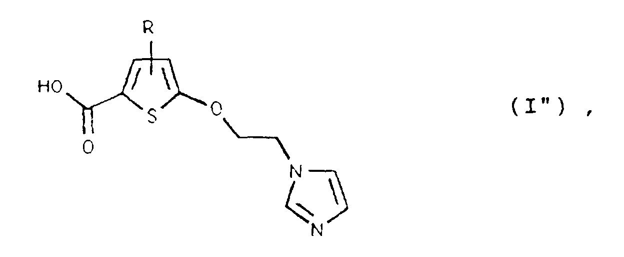 worin R die oben angegebene Bedeutung hat, mit einer Verbindung der allgemeinen Formel (II) 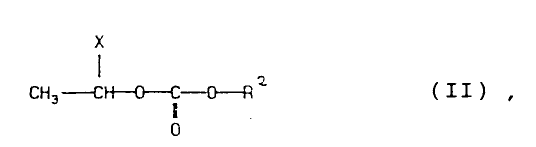 worin X eine zum nukleophilen Austausch geeignete Abgangsgruppe, wie z.B. Halogen, vorzugsweise Chlor oder Brom, darstellt und R² die oben angegebene Bedeutung hat, umgesetzt wird und die erhaltene Verbindung der Formel (I) in ein Additionssalz mit einer schwachen organischen Säure übergeführt wird.

Die Reaktion wird üblicherweise durch Zugabe von mindestens 1 Äquivalent einer starken Base, wie z.B. eines Alkalihydrids oder Alkalicarbonats, zu einer Lösung der Ausgangsverbindung in einem wasserfreien inerten organischen aprotischen Lösungsmittel, wie z.B. Hexamethylphosphorsäuretriamid, Dimethylformamid oder Dimethylsulfoxid, und Zusetzen der Verbindung der Formel (II), vorzugsweise in äquivalenten Mengen oder in geringem Überschuß im gleichen Lösungsmittel, durchgeführt.

Die Reaktion wird bei einer Temperatur im Bereich von Raumtemperatur bis etwa 100°C durchgeführt. Im allgemeinen wird bevorzugt, das Reaktionsgemisch zu erwärmen, z.B. auf 80°C, um die Reaktion zu beschleunigen. Unter diesen Bedingungen ist die Reaktion üblicherweise innerhalb von 2 ½ Stunden abgeschlossen.

Das Reaktionsgemisch wird auf herkömmliche Weise, beispielsweise durch Lösungsmittelextraktion, aufgearbeitet.

Die erfindungsgemäßen Verbindungen der Formel (I) mit einem basischen Imidazolrest können auf übliche Weise in ihre pharmazeutisch annehmbaren Salze mit schwachen organischen Säuren übergeführt werden. Geeignete Säuren sind beispielsweise Fumar-, Oxal-, Malon-, Bernstein-, Adipin-, Malein-, Wein- oder Citronensäure.

Die Herstellung der Ausgangsverbindung wird in der EP-A1-109 381 beschrieben.

Weiters bezieht sich die vorliegende Erfindung auch auf die Anwendung der neuen Verbindungen der Formel (I) allein oder in Mischung mit anderen Wirksubstanzen in Form üblicher oraler galenischer Zusammensetzungen. Die erfindungsgemäßen Verbindungen können in Form von Tabletten oder Kapseln, welche eine Einheitsdosierung der Verbindung zusammen mit Verdünnungsmitteln, wie Maisstärke, Kalziumcarbonat, Dikalziumphosphat, Alginsäure, Lactose, Magnesiumstearat, Primogel oder Talkum enthalten, oral appliziert werden. Die Tabletten werden in herkömmlicher Weise durch Granulieren der Bestandteile und Pressen und die Kapseln durch Einfüllen in Hartgelatinekapseln geeigneter Größe hergestellt.

Für die orale Applikation beim Menschen wird angenommen, daß der tägliche Dosierungswert einer erfindungsgemäßen Verbindung im Bereich von 0,1 bis 20 mg/kg pro Tag für einen typischen erwachsenen Patienten von 70 kg liegt. Daher können Tabletten oder Kapseln üblicherweise 5 bis 150 mg der aktiven Verbindung für die orale Applikation bis zu dreimal am Tag enthalten.

Selbstverständlich wird aber der Arzt in jedem Fall die tatsächliche Dosierung bestimmen, welche für den individuellen Patienten am geeignetsten ist, wobei diese mit dem Alter, der Masse und dem Ansprechen des Patienten variieren kann.

Das folgende Beispiel soll die Erfindung erläutern, ohne sie jedoch einzuschränken.

**Beispiel 1** : Einer Lösung von 5 g (18,20 mMol) 5-[2-(1H-Imidazol-1-yl)-ethoxy]-thiophen-2-carbonsäurehydrochlorid in 100 ml Hexamethylphosphorsäuretriamid (HMPT) werden bei Raumtemperatur unter gutem Rühren 2 g NaH (80 %ige Suspension) portionsweise zugegeben. Dabei steigt die Temperatur auf etwa 40°C an. Zur Salzbildung wird 1 h bei Raumtemperatur gerührt und anschließend eine Lösung von 4,2 g (27,52 mMol) 1-Chlorethylethylcarbonat in 6 ml HMPT bei Raumtemperatur zugetropft.

Das Reaktionsgemisch wird 2 ½ h auf 80°C erhitzt und anschließend zwischen Eiswasser und Ethylacetat (EtOAc) verteilt. Die Phasen werden getrennt, die wässerige Phase dreimal mit EtOAc ausgeschüttelt und die organische Phase zweimal mit einer gesättigten NaHCO₃-Lösung extrahiert. Die EtOAc-Phase wird dreimal mit 2n HCl ausgeschüttelt; die HCl-Phase wird unter Eiskühlung neutralisiert und anschließend mit EtOAc erschöpfend extrahiert.

Nach dem Trocknen über Na₂SO₄ wird filtriert und eingedampft. Es werden 5,78 g gelbes Öl erhalten, das säulenchromatographisch gereinigt wird: Kieselgel, CH₂Cl₂/Ethanol = 20:1. Es werden 4,43 g 5-[2-(1H-Imidazol-1-yl)-ethoxy]-thiophen-2-carbonsäure-1-ethoxycarbonyloxyethylester (68,7 % der Theorie) als blaßgelbes Öl erhalten.

Zur Bildung des Fumarats wird das Öl in wenig Ethanol p.A. gelöst und bei -8°C mit der äquimolaren Menge Fumarsäure (gelöst in Ethanol/Methanol = 6:1) versetzt. Nach mehrstündigem Rühren unter Eiswasserkühlung wird schonend eingedampft und der Rückstand mit eiskaltem Ether zur Kristallisation gebracht. Das erhaltene Fumarat wird aus EtOAc umkristallisiert, wobei 4,0 g 5-[2-(1H-Imidazol-1-yl)-ethoxy]-thiophen-2-carbonsäure-1-ethoxycarbonyloxyethylesterfumarat (62,0 % der Theorie), Fp. 73-75°C, als farblose Kristalle erhalten werden. DC: CH₂Cl₂/Ethanol = 12:1.

| Mikroelementaranalyse: | | | |
|---|---|---|---|
| Berechnet für C₁₉H₂₂N₂O₁₂S | C 48,51; | H 4,71; | N 5,95 %; |
| gefunden | C 48,49; | H 4,59; | N 5,97 %; |

MG = 470,46
¹H-NMR (DMSO): 7,50 (s, 1H, Im-H₂); 7,36; 7,31; 6,11; 6,06 (AB, 2H, Th-H₃ und Th-H₄); 6,87 (s, breit, 2H, Im-H₄ und Im-H₅); 6,68 (q, 1H, -CH-CH₃); 6,55 (s, 2H, -CH=CH-); 4,18 (h, 4H, -OCH₂CH₂-); 3,98 (q, 2H, -OCH₂ -CH₃); 1,37 (d, 3H, CH₃-CH-); 1,08 (t, 3H, -OCH₂-CH₃).

## Patentansprüche

1. Verbindungen der allgemeinen Formel (I) 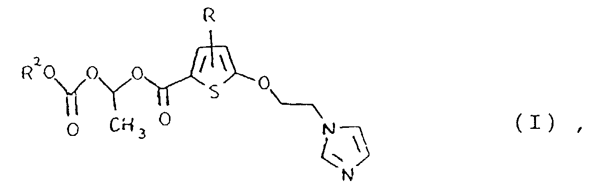 worin R in Stellung 3 oder 4 Wasserstoff, Methyl, Chlor oder Brom darstellt und R² C₁-C₁₀-Alkyl, C₃-C₇-Cycloalkyl oder Benzyl ist, und ihre pharmazeutisch verträglichen Additionssalze mit schwachen organischen Säuren.

2. Verbindung nach Anspruch 1, nämlich 5-[2-(1H-Imidazol-1-yl)-ethoxy]-thiophen-2-carbonsäure-1-ethoxycarbonyloxyethylester.

3. Verbindung nach Anspruch 1, nämlich 5-[2-(1H-Imidazol-1-yl)-ethoxy]-thiophen-2-carbonsäure-1-ethoxycarbonyloxyethylesterfumarat.

4. Verfahren zur Herstellung einer Verbindung nach einem der Ansprüche 1 bis 3, dadurch gekennzeichnet, daß ein Salz einer Verbindung der Formel (I") 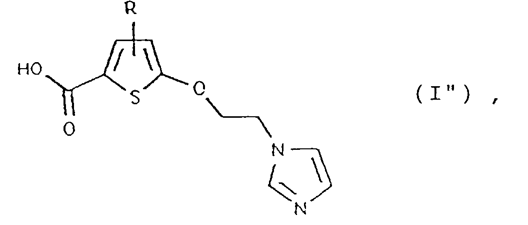 worin R die oben angegebene Bedeutung hat, mit einer Verbindung der allgemeinen Formel (II) 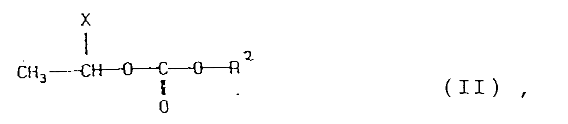 worin X eine zum nukleophilen Austausch geeignete Abgangsgruppe, wie z.B. Halogen, darstellt und R² die oben angegebene Bedeutung hat, umgesetzt wird und die erhaltene Verbindung der Formel (I) in ein Additionssalz mit einer schwachen organischen Säure übergeführt wird.

5. Verfahren nach Anspruch 4, dadurch gekennzeichnet, daß man eine Verbindung (II) einsetzt, worin X Chlor oder Brom ist.

6. Pharmazeutische Zusammensetzung zur oralen Verabreichung, dadurch gekennzeichnet, daß sie eine Verbindung der Formel (I), wie in Anspruch 1 definiert, oder ein pharmazeutisch annehmbares Additionssalz hievon mit einer schwachen organischen Säure zusammen mit einem pharmazeutisch annehmbaren Träger oder Verdünnungsmittel enthält.

7. Verwendung einer Verbindung der Formel (I), wie in Anspruch 1 definiert, als Inhibitor von Thromboxansynthetase.

8. Verwendung einer Verbindung der Formel (I), wie in Anspruch 1 definiert, zur Herstellung von Medikamenten zur Behandlung von durch Thromboxan A₂ verursachten Krankheiten, wie beispielsweise Entzündung, Hypertonie, Thrombus, Gehirnschlag, Asthma, Angina pectoris, ischämische Herzleiden, ischämische Anfälle, Migräne und vaskuläre Komplikationen von Diabetes.

## Claims

1. Compounds of the general formula (I) 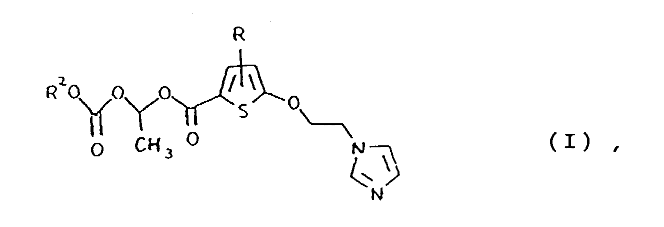 in which R in position 3 or 4 is hydrogen, methyl, chlorine or bromine and R² is C₁-C₁₀-alkyl, C₃-C₇-cycloalkyl or benzyl, and the pharmaceutically acceptable addition salts thereof with weak organic acids.

2. A compound according to claim 1, namely 5-[2-(lH-imidazole-1-yl)-ethoxyl-thiophene-2-carboxylic acid-1-ethoxycarbonyloxyethyl-ester.

3. A compound according to claim 1, namely 5-[2-(lH-imidazole-1-yl)-ethoxyl-thiophene-2-carboxylic acid-1-ethoxycarbonyloxyethyl-ester-fumarate.

4. A process for preparing a compound according to any one of claims 1 to 3, characterized in that a salt of a compound of formula (I") 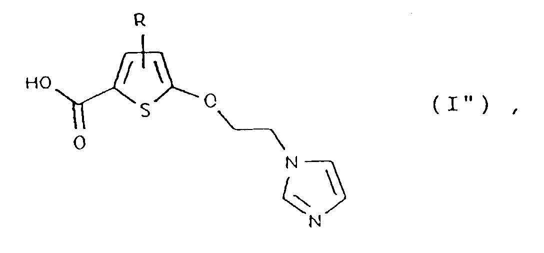 in which R is as defined above, is reacted with a compound of the general formula (II) 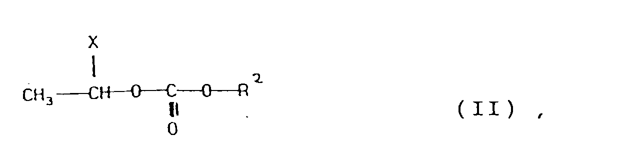 in which X is a leaving group suitable for nucleophilic replacement, such as e.g. halogen, and R² is as defined above and that the obtained compound of formula (I) is converted into an addition salt with a weak organic acid.

5. A process according to claim 4, characterized in that a compound of formula (II) is used, in which X is chlorine or bromine.

6. A pharmaceutical composition for oral administration, characterized in that it comprises a compound of formula (I) as defined in claim 1, or a pharmaceutically acceptable addition salt thereof with a weak organic acid, together with a pharmaceutically acceptable carrier or diluent.

7. The use of a compound of formula (I) as defined in claim 1, as inhibitor of thromboxane synthetase.

8. The use of a compound of formula (I) as defined in claim 1, for the preparation of medicaments for treating diseases caused by thromboxane A₂, such as, e.g., inflammation, hypertension, thrombus, apoplexy, asthma, angina pectoris, ischemic heart disease, ischemic attacks, migraine and vascular complications of diabetes.

## Revendications

1. Composés de formule générale I 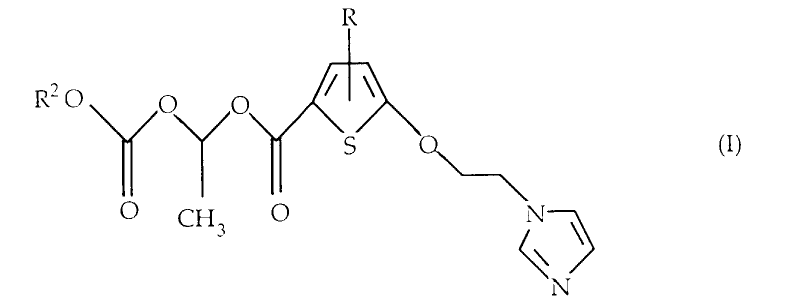 dans laquelle R, en position 3 ou 4, représente l'hydrogène, un groupe méthyle, le chlore ou le brome et R² représente un groupe alkyle en C₁-C₁₀, cycloalkyle en C₃-C₇ ou benzyle, et leurs sels acceptables pour l'usage pharmaceutique formés par addition avec des acides organiques faibles.

2. Composé selon la revendication 1, qui consiste en le 5-[2-(1H-imidazofe-1-yl)-éthoxy]-thiophène-2-carboxylate de 1-éthoxycarbonyloxyéthyle.

3. Composé selon la revendication 1, consistant en le fumarate du 5-[2-(1H-imidazole-1-yl)-éthoxy]-thiophène-2-carboxylate de 1-éthoxycarbonyloxyéthyle.

4. Procédé de préparation d'un composé selon l'une des revendications 1 à 3, caractérisé en ce que l'on fait réagir un sel d'un composé de formule I" 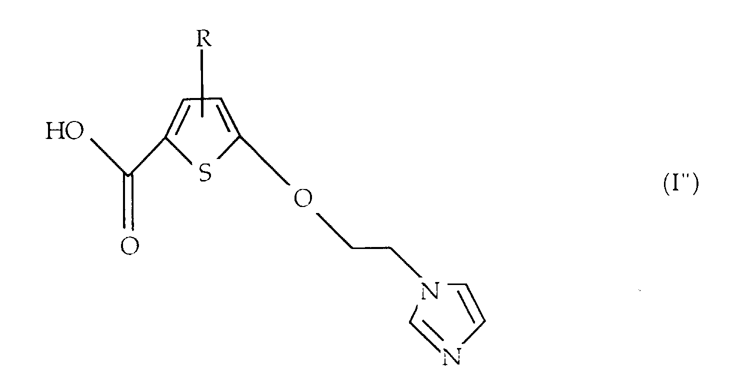 dans laquelle R a les significations indiquées ci-dessus, avec un composé de formule générale II 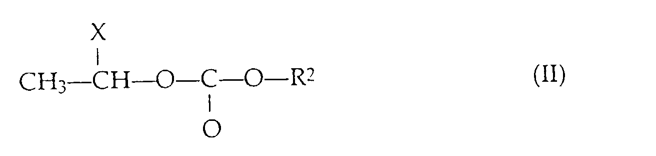 dans laquelle X représente un substituant éliminable approprié à un échange nucléophile, par exemple un halogène, et R² a les significations indiquées ci-dessus, ce qui donne un composé de formule I qu'on convertit en sel par addition avec un acide organique faible.

5. Procédé selon la revendication 4, caractérisé en ce que l'on met en oeuvre un composé II pour lequel X représente le chlore ou le brome.

6. Composition pharmaceutique pour l'administration orale, caractérisée en ce qu'elle contient un composé de formule I selon la revendication 1, ou un sel d'un tel composé acceptable pour l'usage pharmaceutique, formé par addition avec un acide organique faible, avec un véhicule ou diluant acceptable pour l'usage pharmaceutique.

7. Utilisation d'un composé de formule I selon la revendication 1 en tant qu'inhibiteur de la thromboxane synthétase.

8. Utilisation d'un composé de formule I selon la revendication 1 pour la préparation de médicaments conçus pour le traitement des maladies causées par le thromboxane A₂, par exemple les inflammations, les hypertonies, les thromboses, les attaques cérébrales, l'asthme, l'angine de poitrine, les douleurs cardiaques ischémiques, les attaques ischémiques, les migraines et les complications vasculaires du diabète.
